(19) Europäisches Patentamt
European Patent Office
Office européen des brevets

(11) Veröffentlichungsnummer : **0 636 613 A1**

(12) **EUROPÄISCHE PATENTANMELDUNG**

(21) Anmeldenummer : **94890062.6**

(22) Anmeldetag : **31.03.94**

(51) Int. Cl.[6] : **C07D 223/10,** C07D 207/26, C08G 69/44, C08G 69/18

(30) Priorität : **26.07.93 RU 9338121**

(43) Veröffentlichungstag der Anmeldung : **01.02.95 Patentblatt 95/05**

(84) Benannte Vertragsstaaten : **AT BE CH DE DK ES FR GB GR IE IT LI LU NL PT SE**

(71) Anmelder : **Frolov, Vassilii
Teply Stan 8/28
Moskau (RU)**

(72) Erfinder : **Frolov, Vassilii
Teply Stan 8/28
RU-Moskau (RU)**

Erfinder : **Demina, Margarita
Perovskaja Str. 60/32
RU-111394 Moskau (RU)**
Erfinder : **Tchukina, Vera
Novotscherkasski Bld. 42/170
RU-109369 Moskau (RU)**
Erfinder : **Antonenko, Tatjana
Internat. Str. 75b/29
RU-600006 Vladimir 3 (RU)**
Erfinder : **Gladkovski, Gleb, Penkino
Molodjeschnaja Str. 7
RU-601292 Vladimirgebiet (RU)**
Erfinder : **Seiberlich, Feodor
Strasse d.Sieges 17a/58
RU-600000 Vladimir (RU)**

(74) Vertreter : **Kliment, Peter, Dipl.-Ing. Mag.-jur.
Singerstrasse 8/3/8
A-1010 Wien (AT)**

(54) **Alkylphthalate der Polyoxyalkylenphthalyllaktame zur Erhöhung der Schlagzähigkeit und Reissfestigkeit des Polyether-Polykaproamid-Blockkopolymerisates und ihr Herstellungsverfahren.**

(57) Die Erfindung bezieht sich auf Azylderivate der Laktame, die für die Synthese von Polyether-Polykaproamid-Blockkopolymerisaten durch die aktivierte anionische Polymerisation des Kaprolaktams verwendet werden. Die durch Verwendung der vorgeschlagenen Alkylphthalate der Polyoxyalkylenphthalyllaktame erhaltenen Polyether-Polykaproamid-Blockkopolymerisate haben eine hohe Schlagzähigkeit und Reißfestigkeit und können als Konstruktionsmaterial in unterschiedlichen technischen Bereichen eingesetzt werden.

Die Alkylphthalate der Polyoxyalkylenphthalyllaktame nach der Erfindung haben folgende allgemeine Formel :

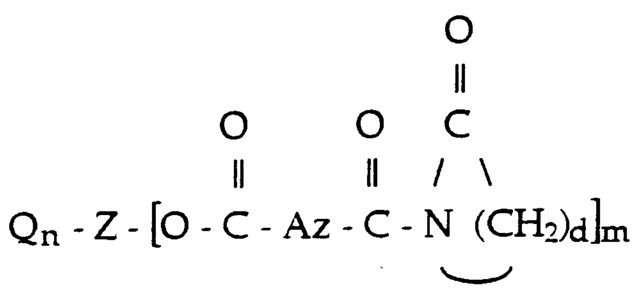

| | |
|---|---|
| Z | Polyoxyalkylenpolyoxyl mit einer Molekularmasse von 2000 bis 6000 mit einer Funktionalität von 2 oder 3 |
| Az | 1,3 - oder 1,4 - Phenylen |
| n | bewegt sich zwischen 0,3 bis 1,5 |
| n+m | ist gleich 2 oder 3 |
| d | ist gleich 5 oder 3 |

EP 0 636 613 A1

$$Q - R - O - \overset{\overset{O}{\|}}{C} - Az - \overset{\overset{O}{\|}}{C} - O -$$

endständige Estergruppen für die Erhöhgung der Schlagzähigkeit und der Reißfestigkeit der Polyether-Polykaproamid-Blockkopolymerisate

R Alkyl mit einer Kohlenstoffanzahl von 1 - 8

Die Erfindung bezieht sich auf Azylderivate der Laktame, die für die Synthese von Polyether-Polykaproamid-Blockkopolymerisat durch die aktivierte anionische Polymerisation des Kaprolaktams verwendet werden. Die durch Verwendung der vorgeschlagenen Alkylphthalate der Polyoxyalkylenphthalyllaktame erhaltenen Polyether-Polykaproamid-Blockkopolymerisate können als Konstruktionsmaterial in unterschiedlichen technischen Bereichen eingesetzt werden.

Die aktivierte anionische Polymerisation der Kaprolaktame, die durch die Salze der Laktame bei Anwesenheit von Aktivatoren - den Azylderivaten der Laktame katalysiert wird, ist bekannt und wird heute für die Erzeugung von Kaprolaktampolymeren, speziell mit der Rekations-Injektions-Formung angewendet. Als Aktivatoren werden oft die Bislaktame von Säuren verwendet (1-4).

Polykaproamide werden dank ihrer hervorragenden physiko-mechanischen Eigenschaften in weiten Bereichen als Konstruktionsmaterialien eingesetzt. Jedoch ist für eine Reihe von Anwendungen eine höhere Schlagzähigkeit des Materials ohne Beeinträchtigung der hohen, für Polykaproamide charakteristischen Festigkeitswerte erforderlich.

Bei Zugabe von hydroxylhältigen Produkten, z.B. von Polyethern (5) in die Reaktionsmasse während der aktivierten anionischen Polymerisation des Kaprolaktames werden diese in die Polymerkette unter Bildung von Laktam-Polyol-Polyazyllaktam-Terpolymeren eingebaut. Die Laktam-Polyol-Polyazyllaktam-Terpolymere haben im Vergleich zu Polykaproamiden eine höhere Schlagzähigkeit und Reißfestigkeit. Neben Polyether kann auch monofunktionaler Alkohol in die Reaktionsmasse eingeführt werden (6).

Diese Methode ist jedoch wenig geeignet für die Herstellung von Erzeugnissen durch die Methode der Reaktions-Injektions-Formung. Die Einführung eines hydroxylhältigen Produktes unmittelbar in die Reaktionsmasse führt zu einer Erhöhung der Reaktionszeit, die im vorliegenden Fall circa 30 Minuten beträgt. Außerdem ist die Einführung eines hydroxylhältigen Produktes technologisch unbequem bei der Herstellung von Erzeugnissen nach der Methode der Reaktions-Injektions-Formung, weil dies die separate Einführung einer großen Anzahl an Komponenten erfordert.

Zur Herstellung der Erzeugnisse aus Polykaproamid, auch mit der Methode der Reaktions-Injektions-Formung, wurde die Verwendung von Polyetherazyllaktamen vorgeschlagen, die sowohl als Aktivatoren als auch als polyetherhältige Komponente dienen (7-10). Die Verwendung solcher oligomerer Polyetherazyllaktame sowie die Einführung von Polyether in die Reaktionsmasse ermöglicht es, die Polyetherblöcke in die Polykaproamidstruktur einzufügen. Im letzten Fall bilden sich infolge der Polymerisation Polyether-Polykaproamid-Blockkopolymerisate, die ähnlich wie Laktam-Polyol-Polyazyllaktam-Terpolymere im Vergleich zu Polykaproamid eine höhere Schlagzähigkeit aufweisen. Die Polymerisationszeit nach dieser Methode beträgt nur 5-10 Minuten; durch die Reduzierung der für die Reaktion erforderlichen Komponenten wird die Technologie zur Produktherstellung vereinfacht.

Durch die Änderung der Struktur des Polyetherblockes im Polyetherazyllaktam, der im Patent (10) als Aktivator der anionischen Polymerisation des Kaprolaktames vorgeschlagen wird, und die Änderung des Polyetherazyllaktam-Anteiles im Polymer ermöglichen eine Einstellung des Schlagzähigkeitsgrades des daraus erhaltenen Polyetherpolykaproamides, wobei sich in allen Fällen mit der Erhöhung der Zähigkeit des Polyetherpolykaproamides seine Reißfestigkeit verringert.

Ziel der vorliegenden Erfindung ist es, neue Produkte zu entwickeln, die sowohl eine hohe Schlagzähigkeit als auch eine hohe Reißfestigkeit besitzen.

Dieses Ziel wird dadurch erreicht, daß neue Verbindungen folgender allgemeiner Formel als Aktivatoren für die anionische Polymerisation von Kaprolaktam verwendet werden:

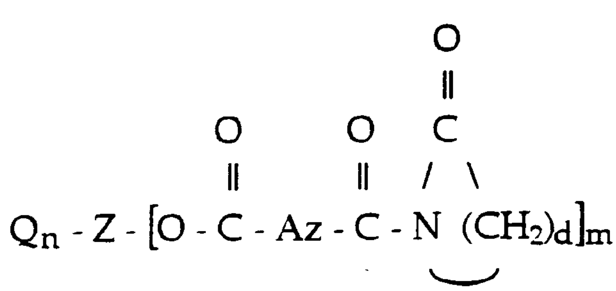

| | |
|---|---|
| Z | Polyoxyalkylenpolyoxyl mit einer Molekularmasse von 2000 bis 6000 mit einer Funktionalität von 2 oder 3 |
| Az | 1,3- oder 1,4-Phenylen |
| n | bewegt sich zwischen 0,3 bis 1,5 |

n+m ist gleich 2 oder 3
d ist gleich 5 oder 3

$$Q-R-O-\overset{\overset{\displaystyle O}{\|}}{C}-Az-\overset{\overset{\displaystyle O}{\|}}{C}-O-$$

endständige Estergruppen für die Erhöhung der Schlagzähigkeit und der Reißfestigkeit der Polyether-Polykaproamid-Blockkopolymerisate

R Alkyl mit einer Kohlenstoffanzahl von 1 - 8

Das vorgeschlagene Produkt stellt ein Alkylphthalat des Polyoxyalkylenphthalyllaktames dar, erhalten durch die Wechselwirkung einer Mischung aus Polyoxyalkylenpolyol, monofunktionalen Alkohols und Laktam mit Phthalsäuredichloranhydrid bei Anwesenheit eines Chlorwasserstoffakzeptors. Dabei muß das Molverhältnis zwischen monofunktionalem Alkohol und Laktam zwischen 0,2 und 1 liegen.

Bei der Verwendung eines solchen Aktivators im Kaprolaktam-Polymerisationsprozeß nehmen seine Azyllaktamgruppen an der Polymerbildung teil während die endständigen Estergruppen unverändert bleiben. Infolgedessen bilden sich in der Polymerstruktur Verzweigungen, die zur Erhöhung seiner Schlagzähigkeit ohne Beeinträchtigung der Reißfestigkeit führen.

Die Schlagzähigkeit des Polyether-Polykaproamid-Blockkopolmeren kann unter Verwendung der Alkylphthalate der Polyoxyalkylenphthalyllaktame, die in der vorliegenden Verbindung als Aktivatoren vorgeschlagen werden, durch Änderung des Verhältnisses zwischen den endständigen Ester- und Azyllaktamgruppen im Aktivator bei ein und derselben Struktur des Polyetherblockes leicht verändert werden. Dabei führt die Erhöhung der Schlagzähigkeit des unter Verwendung des vorgeschlagenen Aktivators hergestellten Polyether-Polykaproamid-Blockkopolmeren zu keiner wesentlichen Verringerung der Reißfestigkeit.

Das Molverhältnis zwischen den endständigen Ester- und Azyllaktamgruppen (n:m) im vorgeschlagenen Aktivator kann sich je nach den erforderlichen physiko-mechanischen Eigenschaften des Polyether-Polykaproamid-Blockkopolmeren ändern, muß jedoch zwischen 0,2 bis 1 liegen.

Das bedeutet zum Beispiel, daß für das Alkylphthalat des Polyoxyalkylenphthalyllaktames, erhalten auf der Basis des dreifunktionalen Polyoxyalkylenes (n+m = 3), mit dem Verhältnis der Estergruppen von 0,5, n = 1 ist.

Wenn dieses Verhältnis geringer als 0,2 ist, wird die Erhöhung der Schlagzähigkeit des Polyether-Polykaproamid-Blockkopolmeren, die mit dem Vorhandensein der Estergruppen zusammenhängt, gering und ein Verhältnis größer als 1 führt wegen der Vergrößerung des Anteiles an nichtaktiven Estergruppen zur Verlangsamung des Kaprolaktam-Polymerisationsprozesses.

Bei Änderung des Verhältnisses zwischen den endständigen Ester- und Azyllaktamgruppen, die als Aktivatoren der Alkylphthalate der Polyoxyalkylenphthalyllaktame vorgeschlagen wurden, von 0,2 bis 1 tritt keine wesentliche Änderung der Erzeugungsgeschwindigkeit von Polyetheramid auf.

Für die in der vorliegenden Erfindung vorgeschlagenen Herstellung der Alkylphthalate der Polyoxyalkylenphthalyllaktame wird folgende Methode vorgeschlagen: Eine Mischung aus Polyoxyalkylenpolyol, monofunktionalem Alkohol und Laktam steht in Wechselwirkung mit dem Phthalsäuredichloranhydrid bei Anwesenheit eines Chlorwasserstoffakzeptors. Dabei muß das Molverhältnis zwischen monofunktionalem Alkohol und Laktam sich zwischen 0,2 und 1 bewegen.

Das oben erwähnte Verhältnis zwischen den chemischen Äquivalenten des monofunktionalen Alkohols und Laktames ist erforderlich zur Herstellung des Alkylphthalates des Polyoxyalkylenphthalyllaktames mit demselben Verhältnis von Ester- und Azyllaktamgruppen.

Der Prozeß wird in einem organischen Lösungsmittel durchgeführt.

In der vorliegenden Erfindung können als Polyoxyalkylenpolyol vielatomige Alkohole dienen, die durch Polyaddition von Propylenoxid, Ethylenoxid und deren Mischungen an Ethylenglykol, Diethylenglykol, Glyzerin und ähnlichen Verbindungen erhalten werden. Das Ethylenoxid-Propylenoxid-Kopolymer kann sowohl eine statistische als auch eine Blockstruktur haben. Die Molekularmasse des Polyols kann zwischen 2000 und 6000 schwanken und die Funktionalität beträgt 2 oder 3. Eine Mischung aus Polyolen ist möglich.

Als Phthalsäuredichloranhydrid kann Terephthalylchlorid oder Isophthalylchlorid verwendet werden.

Als Chlorwasserstoffakzeptor werden tertiäre Amine, zum Beispiel Triethylamin, Trimethylamin, Pyridin oder a- Pikolin verwendet.

Als monofunktionaler Alkohol werden aliphatische Alkohole mit einer Anzahl an Kohlenstoffatomen von 1 bis 8 verwendet.

Als Laktam können Kaprolaktame, Pyrrolidone oder deren Mischungen verwendet werden.

Die Zusammensetzung der erzeugten Produkte wird durch den Masseanteil an gebundenem Laktam bestimmt.

Die Synthese der vorgeschlagenen Alkylphthalate der Polyoxyalkylenphthalyllaktame wurde folgendermaßen durchgeführt:

Beispiel 1:

In einen Kolben mit einem DIN-STARK-Wasserabscheider, einem Thermometer und Tropftrichter wurden 250 g (0,159 Äquivalente) Laprol 5003-2B-10 (Ethylenoxid-Propylenoxid-Blockkopolymerisat mit endständigen Oxyethylgruppen mit einer Funktionalität von 3 (n+m = 3) und einer Molmasse von 5000), 660 ml Toluol und 14,2 g (0,13 Äquivalente) Kaprolaktam (d = 5) gegeben. Der Inhalt wurde bis zum Sieden erhitzt und das Wasser mit dem DIN-STARK-Wasserabscheider abgeschieden, bis sein Masseanteil weniger als 0,2 % betrug. Anschließend wurde der Inhalt auf 10-15°C abgekühlt, 30,5 (0,3 Äquivalente) Terephthalylchlorid (Az-1,4-Phenylen) zugegeben und langsam während 10-15 Minuten aus dem Tropftrichter 39,5 g (0,39 Äquivalente) frisch destilliertes Triethylamin zugeführt. Anschließend wurde 1 Stunde gerührt, der Inhalt auf 100°C erhitzt, weitere 2 Stunden gerührt und 0,9 g (0,03 Äquivalente) Methanol zugegeben. Nach der Zugabe von Methanol wurde noch 1 Stunde bei der gleichen Temperatur gerührt. Dann wurde der Inhalt auf 10-15°C abgekühlt, von den Triethylaminchlorid-Rückständen abfiltriert, im Vakuum das Toluol abdestilliert und somit das oligomere Azyllaktam erhalten. Ausbeute 260 g (90% der Theorie).

Der Masseanteil an gebundenem Laktam wurde durch Ammonolyse des Produktes bestimmt mit nachfolgender gaschromatographischer Bestimmung des ausgeschiedenen Laktames. Gegenüber dem theoretischen Masseanteil an gebundenem Kaprolaktam von 4,9% wurden 4,63% tatsächlich gefunden. Das Molverhältnis zwischen Alkylphthalat- und Azyllaktamgruppen beträgt 0,20 (n = 0,5).

Für die Herstellung des Polyether-Polykaproamid-Kopolymerisates wurde in zwei Gefäße Kaprolaktam bei einer Temperatur von 90-100°C geschmolzen und im ersten Gefäß das Alkylphthalat des Polyoxyalkylenpolyphthalyllaktames in solcher Menge aufgelöst, daß sein Masseanteil 20% der Gesamtmasse des Kaprolaktames in beiden Gefäßen betrug. Im zweiten Gefäß wurde Magnesium-Brom-Kaprolaktam im Verhältnis 1,5 Mol pro Mol Alkylphthalat des Polyoxyalkylenpolyphthalyllaktames gelöst. Nach der Lösung wurde die Temperatur auf 87-90°C angehoben, beide Lösungen schnell unter intensivem Rühren vermischt und die Mischung in eine vorher auf 150°C erwärmte Form mit den Abmessungen 185 x 185 mm und einer Dicke von 4 mm gegossen. Die Aushärtungszeit der Probe war vom Ende des Temperaturanstieges während der Polymerisation abhängig.

Die Schlagzähigkeit wurde nach GOST 4647-80 bestimmt,
die Reißfestigkeit nach GOST 11262-80.

Beispiel 2-5:

Die Herstellung der Alkylphthalate der Polyoxyalkylenpolyphthalyllaktame erfolgte gleich wie in Beispiel 1 mit unterschiedlichen Einsätzen an Methanol und Kaprolaktam. Die Ergebnisse sind in Tabelle 1 zusammengestellt.

**Tabelle 1:**
Bedingungen für die Herstellung und Charakteristik der Produkte aus Beispiel 2-5:

| Beispiel Nr. | Einsatz | | | | Ausbeute | | Massen%* | | Alkylphth./ Azyllakt.** | |
|---|---|---|---|---|---|---|---|---|---|---|
| | Methanol | | Kaprolaktam | | | | berech-net | gefun-den | | |
| | g | (Mol) | g | (Mol) | g | (%) | | | Mol/Mol | n |
| 2 | 1,4 | (0,04) | 12,6 | (0,11) | 253 | (88) | 4,41 | 4,51 | 0,39 | (0,84) |
| 3 | 2,1 | (0,06) | 6,8 | (0,06) | 251 | (90) | 3,00 | 2,88 | 1,00 | (1,5) |
| 4 | 0,6 | (0,02) | 17,0 | (0,15) | 273 | (95) | 5,90 | 6,12 | 0,13 | (0,35) |
| 5 | 2,9 | (0,09) | 6,8 | (0,06) | 257 | (92) | 2,43 | 2,07 | 1,5 | (1,8) |

* Massenanteil des gebundenen Kaprolaktames, %
** Molverhältnis zwischen Alkylphthalat- und Azyllaktamgruppen

Die Beispiele 1-3 zeigen die Möglichkeit für die Herstellung von Alkylphthalaten der

Polyoxyalkylenphthalyllaktame im vorgegebenen Bereich des Verhältnisses zwischen Alkylphthalat- und Azyllaktamgruppen.

Die Beispiele 4 und 5 sind eine Gegenüberstellung.

In Tabelle 2 sind die physiko-mechanischen Eigenschaften der Polyether-Polykaproamid-Blockkopolymerisate dargestellt, die unter Verwendung von den in den Beispielen 1 - 5 angeführten Aktivatoren hergestellt wurden.

**Tabelle 2:**
Physiko-mechanische Eigenschaften der unter Verwendung der in den Beispielen 1-5 angeführten Aktivatoren hergestellten Polyether-Polykaproamid-Blockkopolymerisate

| Beispiel-Nr. | Reißfestigkeit $\sigma_r$, MPa | $a_K$* kJ/m2 | $\tau_{min}$** |
|---|---|---|---|
| 1 | 45 | 25 | 5 |
| 2 | 47 | 31 | 5 |
| 3 | 42 | 35 | 7 |
| 4 | 50 | 20 | 4 |
| 5 | 24 | 41 | 15 |
| lt. Prototyp | 49 | 22 | 4 |

* Schlagzähigkeit nach Sharpi mit Schnitt
** Härtungszeit des Prüfstückes

Die Ergebnisse in Tabelle 2 zeigen, daß mit der Vergrößerung des Anteiles an Alkylphthalatgruppen von 0,2 auf 1 die relative Dehnung und die Schlagzähigkeit ebenfalls steigen, wobei die Reißfestigkeit konstant bleibt.

Zum Vergleich sind die mechanischen Eigenschaften des Polyetheramid-Blockkopolymerisates angeführt, das unter vergleichbaren Bedingungen und unter Verwendung eines laut Prototyp synthetisierten Aktivators hergestellt und geprüft wurde (10).

Wie aus den in Tabelle 2 dargestellten Resultaten ersichtlich ist, führt die Einführung von endständigen Estergruppen in die Struktur des Aktivators im Vergleich zum Prototyp zu einer Erhöhung der relativen Dehnung und der Schlagzähigkeit, wobei die Reißfestigkeit konstant bleibt.

Beispiel 4 zeigt, daß bei einem Ester- und Azyllaktamgruppen-Verhältnis von kleiner als 0,2 keine Erhöhung der Schlagzähigkeit im Vergleich zum Prototyp erfolgt.

Beispiel 5 zeigt, daß bei einem Ester- und Azyllaktamgruppen-Verhältnis von größer als 1 eine Erhöhung der Polymerisationszeit und eine Verringerung der Reißfestigkeit des hergestellten Polyetherpolykaproamides erfolgt.

Beispiel 6:

In den in Beispiel 1 beschriebenen Apparat wurden 582,7 g (0,281 Äquivalente) Laprol 6003-2B-18 (Ethylenoxid-Propylenoxid-Blockkopolymerisat mit endständigen Oxyethylgruppen einer Funktionalität von 3 (n+m=3) und einer Molmasse von 6000), 1200 ml Zyklohexan und 21,1 g (0,19 Äquivalente) Kaprolaktam (d=5) gegeben.

Der Inhalt wurde auf Siedetemperatur erhitzt und das Wasser mittels DIN-STARK-Wasserabscheider abgeschieden, bis sein Masseanteil weniger als 0,02 % betrug. Anschließend wurde der Inhalt auf 10-15°C abgekühlt, 57,0 g (0,56 Äquivalente) Terephthalylchlorid (Az-1,4-Terephthalyl) zugegeben und während 10-15 Minuten aus einem Tropftrichter langsam 68,0 g (0,67 Äquivalente) frischdestilliertes Triethylamin zugetropft. Nach Ende der Zugabe wurde 1 Stunde lang gerührt, auf 100°C erhitzt, weitere 2 Stunden gerührt und 3,3 g (0,1 Äquivalente) Methanol zugegeben. Nach Zugabe des Methanols wurde 1 Stunde lang bei derselben Temperatur gerührt. Dann wurde der Inhalt auf 10-15°C abgekühlt, vom Triethylamin-Rückstand abfiltriert und im Vakuum das Zyklohexan abdestilliert. Die Ausbeute des oligomeren Azyllaktames betrug 575 g (89% der Theorie).

Der theoretische Masseanteil an gebundenem Kaprolaktam beträgt 3,23 %, gefunden wurden 3,68%. Das

Molverhältnis zwischen den Alkylphthalat- und Azyllaktamgruppen betrug 0,50 (n = 1).

Beispiel 7:

Die Herstellungsprozedur erfolgte in Analogie zu Beispiel 1. Zur Herstellung des Aktivators wurden 250 g (0,16 Äquivalente) Laprol 5003-2B-10 mit einer Molmasse von 4600, 600 ml Toluol, 14,5 g (0,13 Äquivalente) Kaprolaktam (d = 5), 32,75 g (0,32 Äquivalente) Isophthalylchlorid (Az-1,3-Terephthalyl), 27,7 g (0,35 Äquivalente) Pyridin und 1,25 g (0,04 Äquivalente) Methanol verwendet. Ausbeute 269 g (93 % der Theorie).

Der theoretische Masseanteil an gebundenem Kaprolaktam beträgt 5,00 %, gefunden wurden 5,53 %. Das Molverhältnis zwischen Alkylphthalat- und Azyllaktamgruppen betrug 0,28 (n = 0,66).

Beispiel 8:

In den in Beispiel 1 beschriebenen Apparat wurden 140 g (0,09 Äquivalente) Laprol 5003-2B-10 (Ethylenoxid-Propylenoxid Blockkopolymerisat mit endständigen 3-funktionalen Oxyethylengruppen (n+m = 3) und einer Molmasse von 4800), 7,0 g (0,06 Äquivalente) Kaprolaktam (d = 5), 1,92 g (0,015 Äquivalente) 2-Ethylhexanol und 320 ml Toluol gegeben. Der Inhalt wurde bis zur Siedetemperatur erhitzt und das Wasser mittels DIN-STARK-Wasserabscheider bis zu einem Masseanteil von weniger als 0,02% abgeschieden. Anschließend wurde der Inhalt bis 10-15°C abgekühlt, 15,8 g (0,15 Äquivalente) Terephthalylchlorid (Az-1,4-Phenylen) und währen 10-15 Minuten aus dem Tropftrichter langsam 19,4 g (0,19 Äquivalente) frischdestilliertes Triethylamin zugegeben. Nach Ende der Zugabe wurde 1 Stunde gerührt, der Inhalt auf 100°C erhitzt und bei dieser Temperatur noch eine Stunde gerührt. Der Inhalt wurde auf 10-15°C abgekühlt, von den Triethylaminchlorid-Rückständen abfiltriert und im Vakuum das Lösungsmittel abdestilliert. Die Ausbeute des oligomeren Azyllaktames betrug 150 g (94% der Theorie).

Der theoretische Massenanteil an gebundenem Kaprolaktam beträgt 3,77 %, gefunden wurden 4,15 %. Das Molverhältnis zwischen Alkylphthalat- und Azyllaktamgruppen betrug 0,25 (n = 0,55).

Beispiel 9:

In den in Beispiel 1 beschriebenen Apparat wurden 130 g (0,13 Äquivalente) Laprol 2102 (Propylenoxidpolymer) mit einer Funktionalität von 2 (n+m = 2) und einer Molmasse von 2000) und 600 ml Toluol gegeben. Der Inhalt wurde auf Siedetemperatur erhitzt und das Wasser mittels DIN-STARK-Wasserabscheider bis zu einem Masseanteil von weniger als 0,02% abgeschieden. Danach wurde der Inhalt auf 10-15°C abgekühlt, 25,82 g (0,20 Äquivalente) Terephthalylchlorid (Az-1,4-Terephthalyl) zugegeben und während 10-15 Minuten aus dem Tropftrichter 15,1 g (0,15 Äquivalente) frischdestilliertes Triethylamin zugetropft. Nach Ende der Zugabe wurde 0,5 Stunden gerührt, 11,9 g (0,10 Äquivalente) Kaprolaktam (d = 5) zugegeben und während 10-15 Minuten noch 15,1 (0,15 Äquivalente) frischdestilliertes Triethylamin zugefügt. Nach Beendigung der Zugabe wurde 1 Stunde gerührt, der Inhalt auf 100°C erhitzt, 0,5 Stunden gerührt und 1,5 g (0,02 Äquivalente) Isopropanol zugefügt. Nach der Zugabe des Isopropanols wurde noch 2 Stunden lang bei derselben Temperatur gerührt. Dann wurde der Inhalt auf 10-15°C abgekühlt, vom Triethylaminchlorid-Rückstand abfiltriert und das Lösungsmittel im Vakuum abdestilliert. Die Ausbeute des oligomeren Azyllaktames betrug 148 g (92% der Theorie).

Der theoretische Masseanteil an gebundenem Kaprolaktam beträgt 7,49 %, gefunden wurden 6,84 %. Das Molverhältnis zwischen Alkylphthalat- und Azyllaktamgruppen betrug 0,20 (n = 0,3).

Beispiel 10:

In den in Beispiel 1 beschriebenen Apparat wurden 253 g (0,16 Äquivalente) Laprol 5003-2B-10 (Ethylenoxid-Propylenoxid Blockkopolymerisat mit endständigen 3-funktionalen Oxyethylengruppen (n+m = 3) und einer Molmasse von 4800), 700 ml Benzol und 9,5 g (0,08 Äquivalente) Kaprolaktam (d = 5) zugegeben. Der Inhalt wurde zum Sieden erhitzt und das Wasser mittels DIN-STARK-Wasserabscheider bis zu einem Masseanteil von weniger als 0,02% abgeschieden. Danach wurde der Inhalt auf 10-15°C abgekühlt, 31,8 g (0,31 Äquivalente) Terephthalylchlorid (Az-1,4-Terephthalyl) und während 10-15 Minuten aus dem Tropftrichter langsam 37,9 g (0,38 Äquivalente) frischdestilliertes Triethylamin zugetropft. Nach Beendigung der Zugabe wurde 1 Stunde lang gerührt, der Inhalt auf 100°C erhitzt, 1 Stunde gerührt und 1,83 g (0,06 Äquivalente) Methanol zugegeben. Nach der Zugabe von Methanol wurde noch 0,5 Stunden bei derselben Temperatur gerührt, 6,03 g (0,07 Äquivalente) Pyrrolidon (d = 3) zugegeben und noch 1 Stunde bei derselben Temperatur gehalten. Anschließend wurde der Inhalt auf 10-15°C abgekühlt, vom Triethylaminchlorid-Rückstand abfiltriert und im Vakuum das Lösungsmittel abdestilliert. Die Ausbeute an oligomerem Azyllaktam betrug 273 g (94 % der Theorie).

Der theoretische Masseanteil an gebundenem Kaprolaktam beträgt 3,26 %, gefunden wurden 3,75 %. Der theoretische Masseanteil an gebundenem Pyrrolidon beträgt 0,55 %, gefunden wurden 0,46 %. Das Molverhältnis zwischen Alkylphthalat- und Azyllaktamgruppen betrug 0,57 (n= 1,1).

Die Beispiele 6-9 zeigen die Möglichkeit der Verwendung verschiedener Polyoxyalkylenpolyole, monofunktionaler Alkohole und Dichloranhydride zur Herstellung der in vorliegender Erfindung vorgeschlagenen Alkylphthalat-Polyoxyalkylenphthalyllaktame auf. In Beispiel 10 wird neben Kaprolaktam auch Pyrrolidon als Laktam verwendet.

In Tabelle 3 sind die physiko-mechanischen Eigenschaften der Polyether-Polykaproamid-Blockkopolymerisate dargestellt, die unter Verwendung der in den Beispielen 6-10 angegebenen Aktivatoren hergestellt wurden. Die Polymerisation wurde in Analogie zu dem in Beispiel 1 verwendeten Aktivator durchgeführt.

**Tabelle 3**

| Physiko-mechanische Eigenschaften der unter Verwendung der in den Beispiele 6-10 angegebenen Aktivatoren hergestellten Polyether-Polykaproamid-Blockkopolymerisate | | | |
|---|---|---|---|
| Beispiel-Nr. | Reißfestigkeit $\sigma_r$, MPa | $a_K$ kJ/m2 | $\tau_{min}$ |
| 6 | 26 | 30 | 7 |
| 7 | 45 | 30 | 5 |
| 8 | 40 | 32 | 8 |
| 9 | 26 | 26 | 6 |
| 10 | 45 | 28 | 3 |

Beispiel 10 (laut Prototyp)

In den in Beispiel 1 beschriebenen Apparat wurden 243 g (0,05 Mol) Laprol 5003-2B-10 (Ethylenoxid-Propylenoxid Blockkopolymerisat mit endständigen 3-funktionalen Oxyethylengruppen und einer Molmasse von 4800) und 400 ml Zyklohexan gegeben. Der Inhalt wurde zum Sieden erhitzt und das Wasser mittels DIN-STARK-Wasserabscheider abgeschieden, wobei sich während 45 Minuten 130 ml Azeotrop ansammelten. Der Inhalt wurde auf 50°C abgekühlt und unter Rühren wurden 31,2 g (0,15 Mol) Terephthalylchlorid zugegeben. Anschließend wurden innerhalb von 10 Minuten 15,3 g (0,15 Mol) Triethylamin in 100 ml Zyklohexan zugefügt. Es wurde auf 50°C abgekühlt, 20 g (0,18 Mol) Kaprolaktam zugegeben und während 20 Minuten eine Lösung von 18 g (0,18 Mol) Triethylamin in 100 ml Zyklohexan zugefügt. Nach Beendigung der Triethylaminzugabe wurde der Inhalt zum Sieden erhitzt und 30 Minuten lang gekocht. Danach wurde der Inhalt auf 15°C abgekühlt, durch eine mit Zyklohexan gewaschene Perliteschicht filtriert und im Vakuum das Lösungsmittel abdestilliert.
Ausbeute: 249 g

Der theoretische Masseanteil an gebundenem Kaprolaktam beträgt 5,88 %, gefunden wurden 6,15 %.

Die Ergebnisse der Tabellen 2 und 3 zeigen, daß alle Alkylphthalat-Polyoxyalkylenphthalyllaktame mit einem in den Beispielen gezeigten Verhältnis zwischen den Ester- und Azyllaktamgruppen von 0,2 bis 1 die Herstellung von Polyether-Polykaproamid-Blockkopolymerisat mit hoher Schlagzähigkeit und Reißfestigkeit erlaubt, wobei diese Materialien dank ihrer kurzen Aushärtungszeit für die Herstellung von Erzeugnissen aus Polyether-Polykaproamid mittels der Reaktions-Injektions-Formung verwendet werden können.

**Stand der Technik:**


1. US Patent 4596855 SA 105:98141
2. US Patent 4346200 SA 97:183488
3. Japanisches Patent 61-258828 SA 106:177046
4. Europatent A2-0113252 kl 008 G 69/18
5. US Patent 4031164 NM? 260/857 MKI 008L 77/00
6. BRD Patent C2-2412106 kl 008G 81/00

7. Europatent B1-0147792 KL 008G 69/20
8. Europatent 067695 KL 008G 69/16
9. Europatent 067694 KL 008G 69/16
10. Europatent 067693 KL 008G 69/16

**Patentansprüche**

1. Alkylphthalate der Polyoxyalkylenphthalyllaktame mit der Formel:

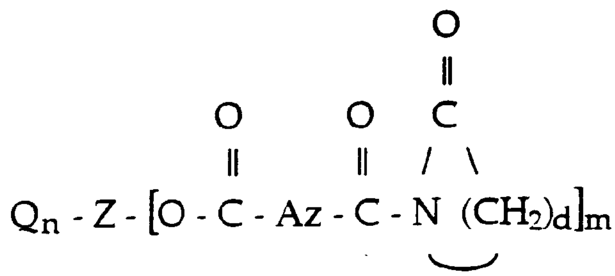

| | |
|---|---|
| Z | Polyoxyalkylenpolyoxyl mit einer Molekularmasse von 2000 bis 6000 mit einer Funktionalität von 2 oder 3 |
| Az | 1,3- oder 1,4- Phenylen |
| n | bewegt sich zwischen 0,3 bis 1,5 |
| n+m | ist gleich 2 oder 3 |
| d | ist gleich 5 oder 3 |

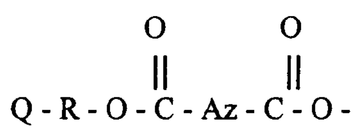

endständige Estergruppen für die Erhöhung der Schlagzähigkeit und der Reißfestigkeit der Polyether-Polykaproamid-Blockkopolymerisate

R Alkyl mit einer Kohlenstoffanzahl von 1 - 8

2. Verfahren zur Herstellung von Alkylphthalaten der Polyoxyalkylenphthalyllaktame nach Anspruch 1, bestehend in der Wechselwirkung zwischen Polyoxyalkylenpolyol, Laktam und monofunktionalem Alkohol mit Phthalsäuredichloranhydrid, wird dadurch charakterisiert, daß das Molverhältnis zwischen monofunktionalem Alkohol und Laktam sich zwischen 0,2 und 1 bewegt.

Europäisches Patentamt

# EUROPÄISCHER RECHERCHENBERICHT

Nummer der Anmeldung
EP 94 89 0062

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | CHEMICAL ABSTRACTS, vol. 90, no. 8, 19. Februar 1979, Columbus, Ohio, US; abstract no. 55737j, H. NISHIZAWA 'N-Acylpolylactams' * Zusammenfassung * & JP-A-78 082 768 (HITACHI CHEMICAL CO. LTD.) --- | 1,2 | C07D223/10<br>C07D207/26<br>C08G69/44<br>C08G69/18 |
| A | EP-A-0 156 079 (SUMITOMO CHEMICAL CO. LTD.) * Ansprüche * --- | 1,2 | |
| A | EP-A-0 189 613 (STAMICARBON B.V.) * Ansprüche * --- | 1,2 | |
| A | EP-A-0 387 895 (N I S PRI VCHTI) * Ansprüche * --- | 1,2 | |
| D,A | CHEMICAL ABSTRACTS, vol. 106, no. 22, 1. Juni 1987, Columbus, Ohio, US; abstract no. 177046c, H. ISEKAWA 'Rigid polyamides with impact resistance' * Zusammenfassung * & JP-A-61 258 828 (MITSUBISHI MONSANTO CHEMICAL CO.) --- | 1,2 | RECHERCHIERTE SACHGEBIETE (Int.Cl.6)<br>C07D<br>C08G |
| D,A | EP-A-0 067 695 (MONSANTO COMPANY) * Ansprüche * --- | 1,2 | |
| A | US-A-4 031 164 (R.M. HEDRICK ET AL.) * Ansprüche * --- | 1,2 | |
| D,A | EP-A-0 113 252 (TORAY INDUSTRIES, INC.) * Ansprüche * ----- | 1,2 | |

Der vorliegende Recherchenbericht wurde für alle Patentansprüche erstellt

| Recherchenort | Abschlußdatum der Recherche | Prüfer |
|---|---|---|
| DEN HAAG | 17. Oktober 1994 | Chouly, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)